# EUROPEAN PATENT APPLICATION

(11) **EP 4 393 428 A1**
(43) Date of publication of application: **03.07.2024**
(21) Application number: 23219754.1
(22) Date of filing: 22.12.2023
(51) Int. Cl.: A61B 18/14, A61B 5/00

(54) **CONTACT FORCE SENSORS FOR BASKET CATHETERS AND METHODS OF USING THEREOF**

(30) Priority: 29.12.2022 US 202263477811 P; 14.11.2023 US 202318508995
(71) Applicant: BIOSENSE WEBSTER (ISRAEL) LTD., 2066717 Yokneam (IL)
(72) Inventor: AGNEW, William James, Irvine, 92618 (US); THALER, Sean D., Irvine, 92618 (US); VAN NIEKERK, Pieter Emmelius, Irvine, 92618 (US); RODRIGUEZ, Jasson, Irvine, 92618 (US)
(74) Representative: Carpmaels & Ransford LLP

(57) **Abstract**

The disclosed technology includes an expandable basket assembly of a medical probe that includes (i) a plurality of spines connected to the distal end of the hub and extending along the longitudinal axis and configured to bow radially outward from the longitudinal axis when the expandable basket assembly is transitioned from a collapsed form to an expanded form, (ii) a plurality of electrodes, each electrode of the plurality of electrodes may be attached to a spine of the plurality of spines, (iii) one or more struts respectively attached to at least one of the plurality of electrodes, and (iv) a force sensor attached to the distal end of the hub and at least one of the plurality of struts. The force sensor may be configured to detect a force applied to one or more of the plurality of electrodes via the one or more struts.

## Description

### CROSS-REFERENCE TO RELATED APPLICATION

This application claims, under 35 U.S.C. § 119(e), priority to and the benefit of U.S. Provisional Patent Application No. 63/477,811, filed December 29, 2022, the entire contents of which are incorporated herein by reference.

### FIELD

The present invention relates generally to medical devices and methods of using medical devices, and in particular basket catheters capable of and methods of detecting a force applied to a basket catheter.

### BACKGROUND

Cardiac arrhythmias, such as atrial fibrillation (AF), occur when regions of cardiac tissue abnormally conduct electrical signals to adjacent tissue. This disrupts the normal cardiac cycle and causes asynchronous rhythm. Certain procedures exist for treating arrhythmia, including surgically disrupting the origin of the signals causing the arrhythmia and disrupting the conducting pathway for such signals. By selectively ablating cardiac tissue by application of energy via a catheter, it is sometimes possible to cease or modify the propagation of unwanted electrical signals from one portion of the heart to another.

Many current ablation approaches in the art utilize radiofrequency (RF) electrical energy to heat tissue. RF ablation can have certain risks related to thermal heating which can lead to tissue charring, burning, steam pop, phrenic nerve palsy, pulmonary vein stenosis, and esophageal fistula.

Cryoablation is an alternative approach to RF ablation that generally reduces thermal risks associated with RF ablation. Maneuvering cryoablation devices and selectively applying cryoablation, however, is generally more challenging compared to RF ablation; therefore cryoablation is not viable in certain anatomical geometries which may be reached by electrical ablation devices.

Some ablation approaches use irreversible electroporation (IRE) to ablate cardiac tissue using nonthermal ablation methods. IRE delivers short pulses of high voltage to tissues and generates an unrecoverable permeabilization of cell membranes. Delivery of IRE energy to tissues using multi-electrode catheters was previously proposed in the patent literature. Examples of systems and devices configured for IRE ablation are disclosed in U.S. Patent Pub. No. 2021/0169550A1, 2021/0169567A1, 2021/0169568A1, 2021/0161592A1, 2021/0196372A1, 2021/0177503A1, and 2021/0186604A1, each of which are incorporated herein by reference and attached in the Appendix of priority application U.S. Provisional Patent Application No. 63/477,811.

Basket catheters are commonly used for mapping or ablating cardiac tissue. Basket catheters generally include a plurality of spines attached to the distal end of the catheter and configured to form a generally spherical shape. Some existing designs of basket catheters include a force sensor disposed between the catheter tube and the basket to sense force applied to the basket. A major limitation of these force sensors, however, is that the force sensor can only detect force applied to the basket as a whole and cannot determine the amount of force applied to each spine or in which direction the force is applied. As will be appreciated, knowing where and in what direction the force is applied to the basket can help a physician more accurately place the basket catheter and ensure sufficient contact is made between the electrodes on the basket catheter and the tissue. What is needed, therefore, are systems and methods for detecting the magnitude and direction of a force applied to various points of the basket catheter.

### SUMMARY

There is provided, in accordance with an example of the disclosed technology, a medical probe. The medical probe may include a hub that may include a distal end and a proximal end and an expandable basket assembly that may include a proximal end, a distal end, and extending along a longitudinal axis. The expandable basket assembly may further include a plurality of spines connected to the distal end of the hub and extending along the longitudinal axis and configured to bow radially outward from the longitudinal axis when the expandable basket assembly is transitioned from a collapsed form to an expanded form. The expandable basket may include a plurality of electrodes, each electrode of the plurality of electrodes may be attached to a spine of the plurality of spines. The expandable basket may include one or more struts respectively attached to at least one of the plurality of electrodes. The expandable basket may also include a force sensor attached to the distal end of the hub and at least one of the plurality of struts. The force sensor may be configured to detect a force applied to one or more of the plurality of electrodes via the one or more struts.

There is provided, in accordance with another example of the disclosed technology, a medical probe. The medical probe may include an expandable basket assembly that may include a proximal end, a distal end, and extending along a longitudinal axis. The expandable basket assembly may include a plurality of spines extending along the longitudinal axis and configured to bow radially outward from the longitudinal axis when the expandable basket assembly is transitioned from a collapsed form to an expanded form. The expandable basket may also include a plurality of electrodes, each electrode of the plurality of electrodes being attached to a spine of the plurality of spines. The expandable basket may also include one or more struts respectively attached to the at least one of the plurality of electrodes. The expandable basket may also include one or more force sensors respectively attached to the one or more struts. Each force sensor may be configured to detect a force applied to a respective electrode attached thereto.

There is provided, in accordance with another example of the disclosed technology, a controller of a medical device. The controller may be configured to receive force data from one or more force sensors of the medical probe, calculate a force applied to the one or more electrodes based at least in part on the received force data, and output the calculated force to a connected display.

### BRIEF DESCRIPTION OF THE DRAWINGS

FIG. 1 is a schematic pictorial illustration of a medical system including a medical probe whose distal end includes a basket assembly with electrodes, in accordance with an embodiment of the present invention;
FIG. 2A is a schematic pictorial illustration showing a perspective view of a medical probe in an expanded form, in accordance with an embodiment of the present invention;
FIG. 2B is a schematic pictorial illustration showing a side view of a medical probe in a collapsed form, in accordance with the disclosed technology;
FIG. 3A is a schematic pictorial illustration showing a perspective view of a medical probe in an expanded form, in accordance with another embodiment of the present invention;
FIG. 3B is a schematic pictorial illustration showing a side view of a medical probe in a collapsed form, in accordance with the another embodiment of the present invention;
FIG. 4 is a schematic pictorial illustration of a contact force sensor showing displacement as the contact force sensor is compressed, in accordance with the disclosed technology;
FIGs. 5A and 5B are schematic pictorial illustrations showing a side view of a spine of a given medical device, in accordance with embodiments of the present invention; and
FIG. 6 is a flowchart illustrating a method of operating a medical probe having a basket assembly and a force sensor, in accordance with an embodiment of the present invention.

### DETAILED DESCRIPTION

The following detailed description should be read with reference to the drawings, in which like elements in different drawings are identically numbered. The drawings, which are not necessarily to scale, depict selected embodiments and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 71% to 110%. In addition, as used herein, the terms "patient," "host," "user," and "subject" refer to any human or animal subject and are not intended to limit the systems or methods to human use, although use of the subject invention in a human patient represents a preferred embodiment. As well, the term "proximal" indicates a location closer to the operator or physician whereas "distal" indicates a location further away to the operator or physician.

As discussed herein, vasculature of a "patient," "host," "user," and "subject" can be vasculature of a human or any animal. It should be appreciated that an animal can be a variety of any applicable type, including, but not limited thereto, mammal, veterinarian animal, livestock animal or pet type animal, etc. As an example, the animal can be a laboratory animal specifically selected to have certain characteristics similar to a human (e.g., rat, dog, pig, monkey, or the like). It should be appreciated that the subject can be any applicable human patient, for example.

As discussed herein, "operator" can include a physician, doctor, surgeon, technician, scientist, or any other individual or delivery instrumentation associated with delivery of a multi-electrode catheter for the treatment of drug refractory atrial fibrillation to a subject.

As discussed herein, the term "ablate" or "ablation", as it relates to the devices and corresponding systems of this disclosure, refers to components and structural features configured to reduce or prevent the generation of erratic cardiac signals in the cells by utilizing non-thermal energy, such as irreversible electroporation (IRE), referred throughout this disclosure interchangeably as pulsed electric field (PEF) and pulsed field ablation (PFA). Ablating or ablation as it relates to the devices and corresponding systems of this disclosure is used throughout this disclosure in reference to non-thermal ablation of cardiac tissue for certain conditions including, but not limited to, arrhythmias, atrial flutter ablation, pulmonary vein isolation, supraventricular tachycardia ablation, and ventricular tachycardia ablation. The term "ablate" or "ablation" also includes known methods, devices, and systems to achieve various forms of bodily tissue ablation as understood by a person skilled in the relevant art.

As discussed herein, the terms "bipolar" and "unipolar" when used to refer to ablation schemes describe ablation schemes which differ with respect to electrical current path and electric field distribution. "Bipolar" refers to ablation scheme utilizing a current path between two electrodes that are both positioned at a treatment site; current density and electric flux density is typically approximately equal at each of the two electrodes. "Unipolar" refers to ablation scheme utilizing a current path between two electrodes where one electrode having a high current density and high electric flux density is positioned at a treatment site, and a second electrode having comparatively lower current density and lower electric flux density is positioned remotely from the treatment site.

As discussed herein, the terms "biphasic pulse" and "monophasic pulse" refer to respective electrical signals. "Biphasic pulse" refers to an electrical signal having a positive-voltage phase pulse (referred to herein as "positive phase") and a negative-voltage phase pulse (referred to herein as "negative phase"). "Monophasic pulse" refers to an electrical signal having only a positive or only a negative phase. Preferably, a system providing the biphasic pulse is configured to prevent application of a direct current voltage (DC) to a patient. For instance, the average voltage of the biphasic pulse can be zero volts with respect to ground or other common reference voltage. Additionally, or alternatively, the system can include a capacitor or other protective component. Where voltage amplitude of the biphasic and/or monophasic pulse is described herein, it is understood that the expressed voltage amplitude is an absolute value of the approximate peak amplitude of each of the positive-voltage phase and/or the negative-voltage phase. Each phase of the biphasic and monophasic pulse preferably has a square shape having an essentially constant voltage amplitude during a majority of the phase duration. Phases of the biphasic pulse are separated in time by an interphase delay. The interphase delay duration is preferably less than or approximately equal to the duration of a phase of the biphasic pulse. The interphase delay duration is more preferably about 25% of the duration of the phase of the biphasic pulse.

As discussed herein, the terms "tubular" and "tube" are to be construed broadly and are not limited to a structure that is a right cylinder or strictly circumferential in cross-section or of a uniform cross-section throughout its length. For example, the tubular structures are generally illustrated as a substantially right cylindrical structure. However, the tubular structures may have a tapered or curved outer surface without departing from the scope of the present disclosure.

The term "temperature rating", as used herein, is defined as the maximum continuous temperature that a component can withstand during its lifetime without causing thermal damage, such as melting or thermal degradation (e.g., charring and crumbling) of the component.

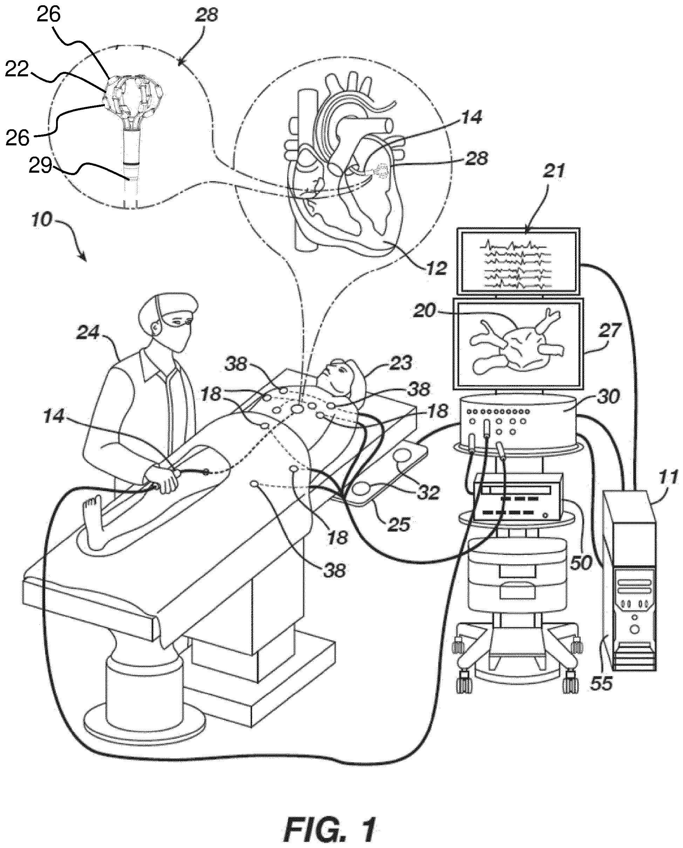

Reference is made to FIG. 1 showing an example catheter-based electrophysiology mapping and ablation system 10. System 10 includes multiple catheters, which are percutaneously inserted by operator 24 through the patient's 23 vascular system into a chamber or vascular structure of a heart 12. Typically, a delivery sheath catheter is inserted into the left or right atrium near a desired location in heart 12. Thereafter, a plurality of catheters can be inserted into the delivery sheath catheter so as to arrive at the desired location. The plurality of catheters may include catheters dedicated for sensing Intracardiac Electrogram (IEGM) signals, catheters dedicated for ablating and/or catheters dedicated for both sensing and ablating. An example catheter 14 that is configured for sensing IEGM is illustrated herein. Operator 24 brings a basket catheter 28 into contact with the heart wall for sensing a target site in heart 12. For ablation, operator 24 would similarly bring a distal end of an ablation catheter to a target site for ablating.

Catheter 14 is an exemplary catheter that includes one and preferably multiple electrodes 26 optionally distributed over a plurality of spines 22 forming a basket assembly 28 at a distal end and configured to sense the IEGM signals. Catheter 14 may additionally include a position sensor 29 embedded in or near the distal tip for tracking position and orientation of basket assembly 28. Optionally and preferably, position sensor 29 is a magnetic based position sensor including three magnetic coils for sensing three-dimensional (3D) position and orientation.

Magnetic based position sensor 29 may be operated together with a location pad 25 including a plurality of magnetic coils 32 configured to generate magnetic fields in a predefined working volume. Real time position of basket assembly 28 of catheter 14 may be tracked based on magnetic fields generated with location pad 25 and sensed by magnetic based position sensor 29. Details of the magnetic based position sensing technology are described in U.S. Patent Nos. 5,391,199; 5,443,489; 5,558,091; 6,172,499; 6,239,724; 6,332,089; 6,484,118; 6,618,612; 6,690,963; 6,788,967; 6,892,091, each of which are incorporated herein by reference and attached in the Appendix of priority application U.S. Provisional Patent Application No. 63/477,811.

System 10 includes one or more electrode patches 38 positioned for skin contact on patient 23 to establish location reference for location pad 25 as well as impedance-based tracking of electrodes 26. For impedance-based tracking, electrical current is directed toward electrodes 26 and sensed at electrode skin patches 38 so that the location of each electrode can be triangulated via the electrode patches 38. Details of the impedance-based location tracking technology are described in US Patent Nos. 7,536,218; 7,756,576; 7,848,787; 7,869,865; and 8,456,182, each of which are incorporated herein by reference and attached in the Appendix of priority application U.S. Provisional Patent Application No. 63/477,811.

A recorder 11 displays electrograms 21 captured with body surface ECG electrodes 18 and intracardiac electrograms (IEGM) captured with electrodes 26 of catheter 14. Recorder 11 may include pacing capability for pacing the heart rhythm and/or may be electrically connected to a standalone pacer.

System 10 may include an ablation energy generator 50 that is adapted to conduct ablative energy to one or more of electrodes at a distal tip of a catheter configured for ablating. Energy produced by ablation energy generator 50 may include, but is not limited to, radiofrequency (RF) energy or pulsed-field ablation (PFA) energy, including monopolar or bipolar high-voltage DC pulses as may be used to effect irreversible electroporation (IRE), or combinations thereof. The signals may be biphasic or monophasic.

Patient interface unit (PIU) 30 is an interface configured to establish electrical communication between catheters, electrophysiological equipment, power supply and a workstation 55 for controlling operation of system 10. Electrophysiological equipment of system 10 may include for example, multiple catheters, location pad 25, body surface ECG electrodes 18, electrode patches 38, ablation energy generator 50, and recorder 11. Optionally and preferably, PIU 30 additionally includes processing capability for implementing real-time computations of location of the catheters and for performing ECG calculations.

Workstation 55 includes memory, processor unit with memory or storage with appropriate operating software loaded therein, and user interface capability. Workstation 55 may provide multiple functions, optionally including (1) modeling the endocardial anatomy in three-dimensions (3D) and rendering the model or anatomical map 20 for display on a display device 27, (2) displaying on display device 27 activation sequences (or other data) compiled from recorded electrograms 21 in representative visual indicia or imagery superimposed on the rendered anatomical map 20, (3) displaying real-time location and orientation of multiple catheters within the heart chamber, and (5) displaying on display device 27 sites of interest such as places where ablation energy has been applied. One commercial product embodying elements of the system 10 is available as the CARTO^{™} 3 System, available from Biosense Webster, Inc., 31 Technology Drive, Suite 200, Irvine, CA 92618, USA.

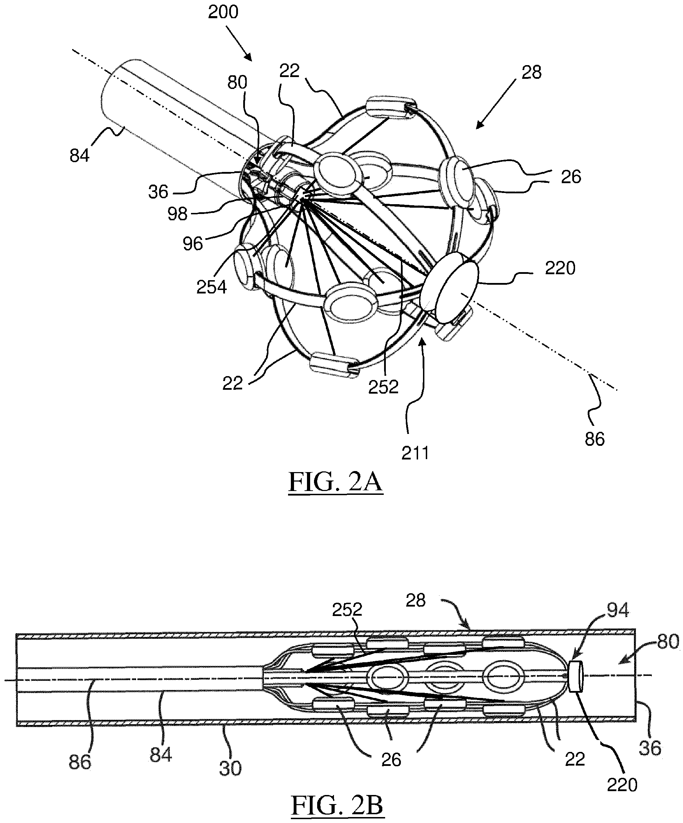

FIG. 2A is a schematic pictorial illustration showing a perspective view of a medical probe 200 having a basket assembly 28 in an expanded form when unconstrained, such as by being advanced out of a tubular shaft lumen 80 at a distal end 36 of a tubular shaft 82. FIG. 2B shows the basket assembly in a collapsed form within tubular shaft 82. In the expanded form (FIG. 2A), the spines 22 bow radially outwardly along a longitudinal axis 86 and in the collapsed form (FIG. 2B) the spines are constrained generally along the longitudinal axis 86 of tubular shaft 82.

As shown in FIG. 2A, basket assembly 28 includes a plurality of flexible spines 22 that are formed at the end of a tubular shaft 84 and are connected at both ends. During a medical procedure, operator 24 can deploy basket assembly 28 by extending tubular shaft 84 from tubular shaft 82 causing the basket assembly 28 to exit the tubular shaft 84 and transition to the expanded form. Spines 22 may have elliptical (e.g., circular) or rectangular (that may appear to be flat) cross-sections, and include a flexible, resilient material (e.g., a shape-memory alloy such as nickel-titanium, also known as Nitinol) forming a strut as will be described in greater detail herein.

In examples described herein, electrodes 26 can be configured to deliver ablation energy (RF and/or IRE) to tissue in heart 12. In addition to using electrodes 26 to deliver ablation energy, the electrodes can also be used to measure a physiological property such as local surface electrical potentials at respective locations on tissue in heart 12. The electrodes 26 can be biased such that a greater portion of the electrode 26 faces outwardly from the basket assembly 39 such that the electrodes 26 deliver a greater amount of electrical energy outwardly away from the basket assembly 28 (i.e., toward the heart 12 tissue) than inwardly toward the basket catheter 38.

Examples of materials ideally suited for forming electrodes 26 include gold, platinum, and palladium (and their respective alloys). These materials also have high thermal conductivity which allows the minimal heat generated on the tissue (i.e., by the ablation energy delivered to the tissue) to be conducted through the electrodes to the back side of the electrodes (i.e., the portions of the electrodes on the inner sides of the spines), and then to the blood pool in heart 12.

Basket assembly 28 can include a stem 96 that extends longitudinally from a distal end 36 of shaft 84 towards distal end 94 of basket assembly 28. The disclosed technology can include an irrigation system that delivers irrigation fluid to spray ports 98. For example, stem 96 can include multiple spray ports 98, wherein each given spray port 98 can be angled to aim delivery of the irrigation fluid to either a given electrode 26 or to tissue in heart 12. The electrodes 26 can be cooled by aiming the irrigation fluid, via spray ports 98, at the portion of the electrodes 26 on the inner side of the spines 22.

The basket assembly 28 can include a central intersection 211 at a point where the spines 22 converge near the distal end 94. The basket assembly 28 can include contact force sensor assembly 220 attached to the central intersections 211. By attaching the contact force sensor assembly 220 to the central intersection 211, the contact force sensor assembly 220 can be configured to detect a force applied to the basket assembly 28 at the distal end 94. In this way, the contact force sensor assembly 220 can be configured to more easily detect a force applied to the basket assembly 28 (e.g., when the basket assembly 28 contacts tissue).

The contact force sensor assembly 220 can be or include any suitable type of contact force sensor for the application. For example, the contact force sensor assembly 220 can be a load cell, a strain gauge, a piezoelectric sensor, a force sensing resistor, a magnetic force sensor, etc.

Additionally or alternatively, the basket assembly 28 may include one or more struts 252 that may be respectively attached to at least one of the plurality of electrodes 26. The basket assembly 28 may also include an electrode force sensor 254 attached to the distal end of the hub or stem 96. The electrode force sensor 254 may be configured to detect a force applied to one or more of the plurality of electrodes 26 via the one or more struts 252. Put another way, a force applied to one or more of the electrodes 26 (e.g., due to one or more electrodes 26 contacting tissue) may be transferred to the electrode force sensor 254 by corresponding struts 252 which may slightly contract, bend, or otherwise change form as a force is applied to corresponding electrodes 26 but otherwise transfer the force from one end to another. The one or more struts may include a biocompatible material selected from a group consisting essentially of nitinol, cobalt chromium, stainless steel, titanium, or combinations thereof. The electrode force sensor 254 may include a strain gauge, a piezoelectric sensor, a force sensing resistor, a magnetic force sensor, etc.

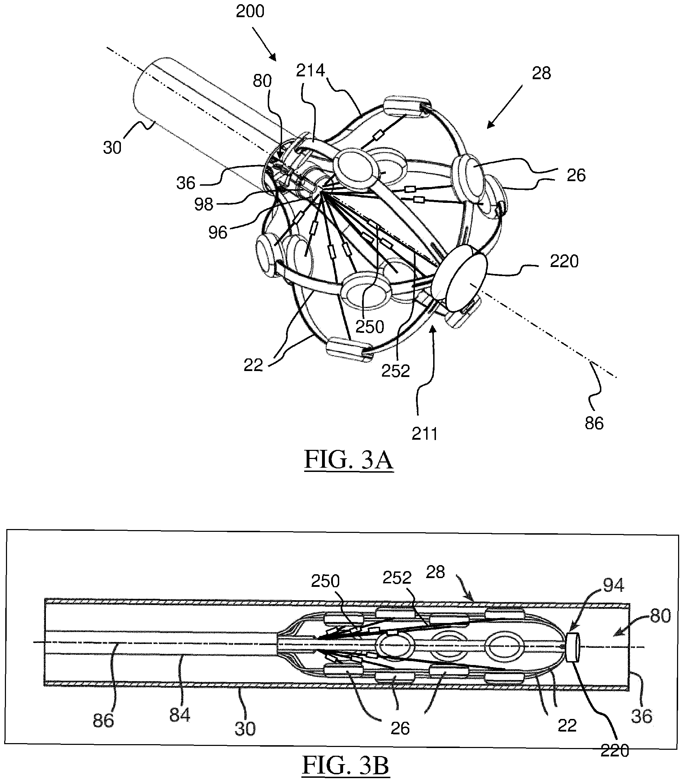

As shown in FIGs. 3A, and 3B, one or more force sensors 250 may be respectively attached to one or more struts 252, which may be respectively attached to one or more electrodes 26 and the hub or stem 96. The one or more force sensors 250 may each comprise a strain gauge that may be attached to an inwardly facing portion of a strut 252 or an outwardly facing portion of a struct 252. Regardless, the one or force sensors 250 may sense when a force is applied to an attached electrode 26 by detecting movement (e.g., contracting, bending, or other movement) of the corresponding strut 252 due to the application of a force at its end.

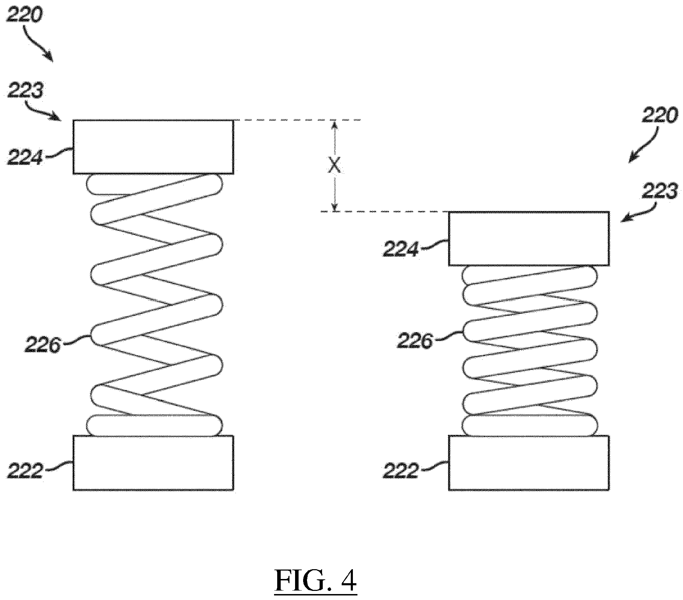

As one non-limiting example illustrated in FIG. 4, the contact force sensor assembly 220 can include a proximal end 223 and a distal end 224. The proximal end 222 can house a magnetic field generator coil and the distal end 224 can house a magnetic field sensor, or vice versa. As will be appreciated, the magnetic field generator coil can be configured to generate a magnetic field while the magnetic field sensor can be configured to detect the presence and magnitude of the magnetic field.

The contact force sensor assembly 220 can further include a deflection portion 226 disposed between the proximal end 222 and the distal end 224. The deflection portion 226 can be configured to deflect when a force is applied to the contact force sensor assembly 220. In other words, the deflection portion 226 can be configured to permit the proximal end 222 and the distal end 224 of the contact force sensor assembly 220 to move closer to each other when a force is applied to the contact force sensor assembly 220. To illustrate, FIG. 4 shows a change in position "X" when the deflection portion 226 is compressed as a force is applied to the contact force sensor 220. As will be appreciated, the change in position "X" increases (or be a greater distance) when the deflection portion 226 is compressed to a greater extent.

In one example, the deflection portion 226 is a spring positioned between the proximal end 222 and the distal end 224. As another example, the deflection portion 226 can comprise a helical spring formed into a body of the contact force sensor assembly 220. For example, helical cuts can be made in the body of the contact force sensor assembly 220 to form a helical spring. In this way, the body of the contact force sensor assembly 220 can itself form a spring without the need for additional components.

As will be appreciated, when the distal end 224 is moved closer to the proximal end 222 when a force is applied to the contact force sensor assembly 220, the magnetic field sensor can detect a change in the magnitude of the force of the magnetic field generated by the magnetic field generator coil. Because the spring constant K of the deflection portion 226 can be predetermined and the distance between the magnetic field generator coil and the magnetic field sensor can be detected as the distal end 224 is brought closer to the proximal end 222, the force applied to the basket catheter 28 can be determined (e.g., by using Hooke's law, or the equation F=d*K). Furthermore, the contact force sensor assembly 220 can receive electrical signals from, and provides electrical signals to, workstation 55, to process received signals and determine forces, e.g., sub-gram forces, exerted on the basket assembly 28.

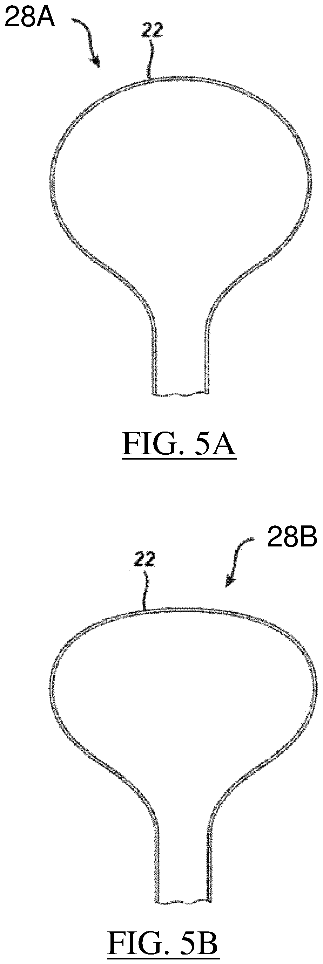

FIGs. 5A and 5B are schematic pictorial illustrations showing a side view of a spine 22 of a given basket catheter 28, in accordance with examples of the disclosed technology. As will be appreciated, the spine 22 illustrated in FIGS. 5A and 5B is a single spine 22 and can be representative of the plurality of spines 22 of the basket assembly 28 described herein. In other words, the plurality of spines 22 forming the basket assembly 28 can each be configured to form the same or similar shape when in the expanded form such that the plurality of spines 22 together form a desired shape. To illustrate, the spine 22 as shown in FIG. 5A can be configured to form an approximately circular shape when in the expanded form. Thus, when combined with other spines 22 to form the basket assembly 28, the plurality of spines 22 can be configured to form an approximately spherical shape when the basket assembly 28 is in the expanded form. As another example, the spine 22 shown in FIG. 5B can be configured to form an approximately elliptical shape when in the expanded form. Thus, when combined with other spines 22 to form the basket assembly 28, the plurality of spines 22 can be configured to form an approximately oblate-spheroid shape when the basket assembly 28 is in the expanded form. Although not every variation of shape is shown or described herein, one skilled in the art will appreciate that the spines 22 can be further configured to form other various shapes as would be suitable for the particular application.

By including spines 22 configured to form various shapes when in the expanded form, the basket assembly 28 can be configured to position the various electrodes 26 attached to the spines 22 at various locations, with each location being nearer or farther from the distal end of the flexible tubular shaft 82. For example, an electrode 26 attached to the spine 22 illustrated in FIG. 5A near the middle of the spine 22 would be farther from the distal end of the flexible tubular shaft 82 than the spine 22 illustrated in FIG. 5B when the basket assembly 28 is in the expanded form.

The workstation 55 can be calibrated to detect the magnitude and direction of the force applied to the basket catheter 28 depending on which shape the basket 28 is configured to take when fully expanded. For example, as will be appreciated, as basket catheter 28 having a more spherical shape will exhibit slightly different characteristics as it deflects when a force is applied than a basket catheter 28 having a more oblate-spheroid shape. Thus, by calibrating the workstation 55 to analyze the data received from the force sensors 250, 254 dependent on the shape of the basket catheter 28, the workstation 55 can more accurately determine the magnitude and direction of the force applied to the basket catheter 28.

The disclosed technology can further include an electrically resistive jacket disposed over the spines 22 to electrically isolate the spines 22 from the electrodes 26. In this way, the electrodes 26 can be prevented from forming a short circuit to the spine 22.

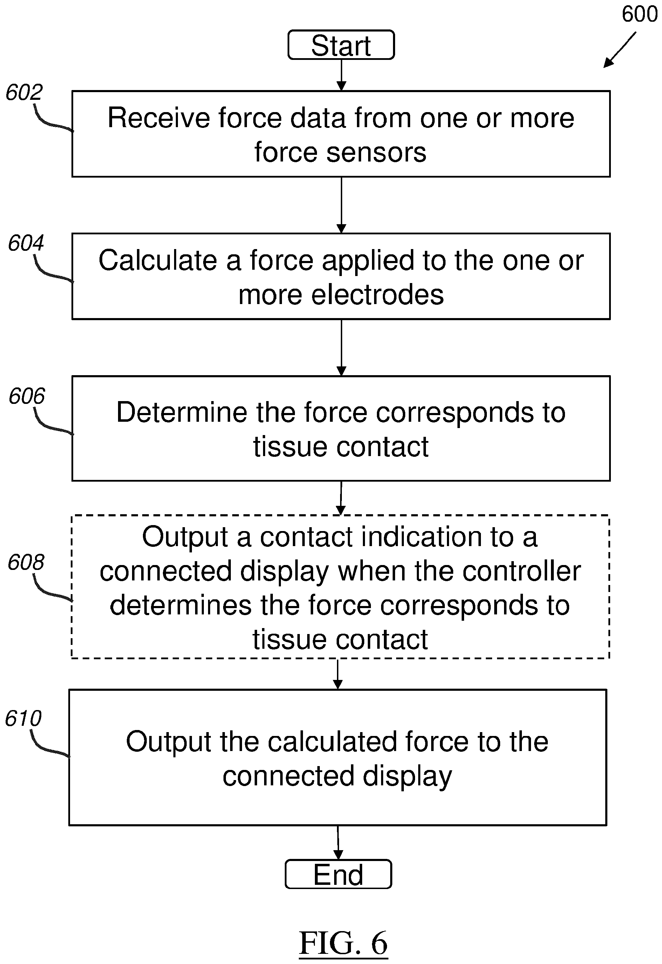

FIG. 6 is a flowchart illustrating a method 600 of operating a medical probe, in accordance with an embodiment of the present invention. The method 600 can include aligning receiving 602 force data from one or more force sensors (e.g., force sensors 254) attached to a spine 22 and/or hub or stem 96 of a basket catheter (e.g., basket catheter 28). The method 600 can include calculating 604 a force applied to the one or more electrodes 26. For example, the method 600 can include determining a magnitude and a direction of a force applied to the basket catheter. The method 600 can further include determining 606 the calculated force corresponds to tissue contact by comparing the calculated force to a predetermined threshold that corresponds to tissue contact. The method 600 may further include outputting 608 a contact indication to a connected display when the controller determines the force corresponds to tissue contact. The method 600 may further include outputting 610 the calculated force to the connected display.

As will be appreciated by one skilled in the art, the method 600 can include any of the various features of the disclosed technology described herein and can be varied depending on the particular configuration. That is, the method 600 can be varied to include any of the features described in relation to basket catheter 28.

The disclosed technology described herein can be further understood according to the following clauses:
Clause 1: A medical probe, comprising: a hub comprising a distal end and a proximal end; an expandable basket assembly comprising a proximal end, a distal end, and extending along a longitudinal axis, the expandable basket assembly comprising; a plurality of spines connected to the distal end of the hub and extending along the longitudinal axis and configured to bow radially outward from the longitudinal axis when the expandable basket assembly is transitioned from a collapsed form to an expanded form; a plurality of electrodes, each electrode of the plurality of electrodes being attached to a spine of the plurality of spines; one or more struts respectively attached to at least one of the plurality of electrodes; and a force sensor attached to the distal end of the hub and at least one of the plurality of struts, the force sensor configured to detect a force applied to one or more of the plurality of electrodes via the one or more struts.
Clause 2: The medical probe according to Clause 1, wherein each of the one or more struts comprise have a first width less than a second width of each of the plurality of spines.
Clause 3: The medical probe according to Clause 1, wherein the force sensor comprises a strain gauge.
Clause 4: The medical probe according to Clause 1, wherein each spine of the plurality of spines comprises nitinol, cobalt chromium, stainless steel, titanium, or combinations thereof.
Clause 5: The medical probe according to Clause 1, wherein the plurality of spines is configured to form an approximately spherically-shaped basket assembly when in the expanded form.
Clause 6: The medical probe according to Clause 1, wherein the plurality of spines is configured form an approximately oblate-spheroid basket assembly when in the expanded form.
Clause 7: The medical probe according to Clause 1, wherein the hub further comprising spray ports configured to deliver an irrigation fluid to the plurality of electrodes.
Clause 8: The medical probe according to Clause 1, further comprising a plurality of electrically insulative jackets each disposed between a respective spine of the plurality of spines and a respective electrode of the plurality of electrodes, thereby electrically isolating the plurality of electrodes from the plurality of spines.
Clause 9: A medical probe, comprising: an expandable basket assembly comprising a proximal end, a distal end, and extending along a longitudinal axis, the expandable basket assembly comprising: a plurality of spines extending along the longitudinal axis and configured to bow radially outward from the longitudinal axis when the expandable basket assembly is transitioned from a collapsed form to an expanded form; a plurality of electrodes, each electrode of the plurality of electrodes being attached to a spine of the plurality of spines; one or more struts respectively attached to the at least one of the plurality of electrodes; and one or more force sensors respectively attached to the one or more struts, each force sensor configured to detect a force applied to a respective electrode attached thereto.
Clause 10: The medical probe according to Clause 9, wherein each of the plurality of force sensors comprises a strain gauge.
Clause 11: The medical probe according to Clause 10, wherein each strain gauge is attached to an inwardly-facing surface of a strut.
Clause 12: The medical probe according to Clause 10, wherein each strain gauge is attached to an outwardly-facing surface of the strut.
Clause 13: The medical probe according to Clause 9, wherein each of the one or more struts comprise a biocompatible material selected from a group consisting essentially of nitinol, cobalt chromium, stainless steel, titanium, or combinations thereof.
Clause 14: The medical probe according to Clause 9, wherein the plurality of spines is configured to form an approximately spherically-shaped basket assembly when in the expanded form.
Clause 15: The medical probe according to Clause 9, wherein the plurality of spines is configured form an approximately oblate-spheroid basket assembly when in the expanded form.
Clause 16: The medical probe according to Clause 9, further comprising a hub comprising a distal end connected to a proximal end of the plurality of spine and spray ports configured to deliver an irrigation fluid to the plurality of electrodes.
Clause 17: The medical probe according to Clause 9, further comprising a plurality of electrically insulative jackets each disposed between a respective spine of the plurality of spines and a respective electrode of the plurality of electrodes, thereby electrically isolating the plurality of electrodes from the plurality of spines.
Clause 18: A controller of a medical device, the controller configured to: receive force data from one or more force sensors of the medical probe of Clauses 1 or 9; calculate a force applied to the one or more electrodes based at least in part on the received force data; and output the calculated force to a connected display.
Clause 19: The controller of the medical device of Clause 18, wherein the controller is further configured to: determine calculated force corresponds to tissue contact; and output a contact indication to the connected display when the controller determines the force corresponds to tissue contact.

The embodiments described above are cited by way of example, and the present invention is not limited by what has been particularly shown and described hereinabove. Rather, the scope of the invention includes both combinations and sub combinations of the various features described hereinabove, as well as variations and modifications thereof which would occur to persons skilled in the art upon reading the foregoing description and which are not disclosed in the prior art.

## Claims

1. A medical probe, comprising:
a hub comprising a distal end and a proximal end;
an expandable basket assembly comprising a proximal end, a distal end, and extending along a longitudinal axis, the expandable basket assembly comprising:
a plurality of spines connected to the distal end of the hub and extending along the longitudinal axis and configured to bow radially outward from the longitudinal axis when the expandable basket assembly is transitioned from a collapsed form to an expanded form;
a plurality of electrodes, each electrode of the plurality of electrodes being attached to a spine of the plurality of spines;
one or more struts respectively attached to at least one of the plurality of electrodes; and
a force sensor attached to the distal end of the hub and at least one of the plurality of struts, the force sensor configured to detect a force applied to one or more of the plurality of electrodes via the one or more struts.

2. The medical probe according to Claim 1, wherein each of the one or more struts comprise have a first width less than a second width of each of the plurality of spines.

3. The medical probe according to Claim 1, wherein the force sensor comprises a strain gauge.

4. The medical probe according to Claim 1, wherein each spine of the plurality of spines comprises nitinol, cobalt chromium, stainless steel, titanium, or combinations thereof.

5. The medical probe according to Claim 1, wherein the hub further comprising spray ports configured to deliver an irrigation fluid to the plurality of electrodes.

6. A medical probe, comprising:
an expandable basket assembly comprising a proximal end, a distal end, and extending along a longitudinal axis, the expandable basket assembly comprising:
a plurality of spines extending along the longitudinal axis and configured to bow radially outward from the longitudinal axis when the expandable basket assembly is transitioned from a collapsed form to an expanded form;
a plurality of electrodes, each electrode of the plurality of electrodes being attached to a spine of the plurality of spines;
one or more struts respectively attached to the at least one of the plurality of electrodes; and
one or more force sensors respectively attached to the one or more struts, each force sensor configured to detect a force applied to a respective electrode attached thereto.

7. The medical probe according to Claim 6, wherein each of the plurality of force sensors comprises a strain gauge.

8. The medical probe according to Claim 7, wherein each strain gauge is attached to an inwardly-facing surface of a strut.

9. The medical probe according to Claim 7, wherein each strain gauge is attached to an outwardly-facing surface of the strut.

10. The medical probe according to Claim 6, wherein each of the one or more struts comprise a biocompatible material selected from a group consisting essentially of nitinol, cobalt chromium, stainless steel, titanium, or combinations thereof.

11. The medical probe according to Claim 1 or Claim 6, wherein the plurality of spines is configured to form an approximately spherically-shaped basket assembly or an approximately oblate-spheroid basket assembly when in the expanded form.

12. The medical probe according to Claim 6, further comprising a hub comprising a distal end connected to a proximal end of the plurality of spine and spray ports configured to deliver an irrigation fluid to the plurality of electrodes.

13. The medical probe according to Claim 1 or Claim 6, further comprising a plurality of electrically insulative jackets each disposed between a respective spine of the plurality of spines and a respective electrode of the plurality of electrodes, thereby electrically isolating the plurality of electrodes from the plurality of spines.

14. A controller of a medical device, the controller configured to:
receive force data from one or more force sensors of the medical probe of Claim 1;
calculate a force applied to the one or more electrodes based at least in part on the received force data; and
output the calculated force to a connected display.

15. The controller of the medical device of Claim 14, wherein the controller is further configured to:
determine calculated force corresponds to tissue contact; and
output a contact indication to the connected display when the controller determines the force corresponds to tissue contact.
